# EUROPEAN PATENT SPECIFICATION

(11) **EP 1 146 890 B1**
(45) Date of publication and mention of the grant of the patent: **22.04.2009**
(21) Application number: 00907063.2
(22) Date of filing: 28.01.2000
(51) Int. Cl.: A61K 38/16, A61K 9/12, A61K 47/18

(54) **BUCCAL DELIVERY SYSTEM FOR PROTEINACEOUS MEDICAMENTS**
BUKKALES VERABREICHUNGSSYSTEM FÜR PROTEINHALTIGE MEDIKAMENTE
SYSTEME D'ADMINISTRATION ORALE DE MEDICAMENTS PROTEINIQUES

(30) Priority: 29.01.1999 US 240455
(43) Date of publication of application: 24.10.2001
(73) Proprietor: Esperanza Peptide, Ltd., Ayrshire KA30 9JY (GB)
(72) Inventor: MUNDSCHENK, David, D., Dania, FL 33004 (US)
(74) Representative: Thom, Russell
(86) International application number: PCT/US2000/002210
(87) International publication number: WO 2000/044393

(56) References cited:
- EP-A- 0 327 756
- EP-A- 0 550 031
- WO-A-97/43407
- GB-A- 2 127 689
- SIEGEL I A ET AL: "EFFECTS OF SURFACTANTS ON THE PERMEABILITY OF CANINE ORAL MUCOSA IN-VITRO" TOXICOLOGY LETTERS (AMSTERDAM), vol. 26, no. 2-3, 1985, pages 153-158, XP000938712 ISSN: 0378-4274
- HOPPER P.E. ET AL: 'SERUM GLUCOSE AND INSULIN RESPONSES TO AN INSULIN-CONTAINING OPHTHALMIC SOLUTION ADMINISTERED TOPICALLY IN CLINICALLY NORMAL CATS' AMERICAN JOURNAL OF VETERINARY RESEARC vol. 52, no. 6, 1991, pages 903 - 907, XP009036207

## Description

### TECHNICAL FIELD

The present invention relates to methods and systems for delivering medicaments to the body, and in particular, to mucosal membranes and surfaces. In another aspect, the invention relates to medicament and other formulations that include the use of quaternary ammonium salts, such as benzalkonium chloride. In yet another aspect, the invention relates to the delivery to the body of inactivated bioactive peptides.

### BACKGROUND OF THE INVENTION

A variety of approaches have been described for use in delivering medicaments to the body, including intravaneously, orally, and topically. Several of such approaches involve nasooral delivery, including delivery to mucosal surfaces by the use of sprays, tablets, devices and the like.

The mouth, also known as the oral or buccal cavity, is placed at the start of the alimentary canal. Anatomically, Gray's Anatomy describes the mouth as consisting of two parts, including an outer, smaller portion, the vestibule (vestibulum oris), and an inner, larger part, the cavity proper (cavum oris proprium). The vestibule is the slit-like aperture bounded in front and laterally by the lips and cheeks, and behind and internally by the gums and teeth. Above and below, the vestibule is limited by the reflection of the mucous membrane from the lips and cheeks, to the gums covering the upper and lower alveolar arch, respectively. The vestibule receives the secretion from the parotid glands and communicates, when the jaws are closed, with the cavity of the mouth by an aperture on each side behind the wisdom teeth.

The cavum oris proprium is bounded laterally and in front by the alveolar arches with their contained teeth, and behind it communicates with the pharynx by a constricted aperture (isthmus faucuim). It is roofed by the hard and soft palate, while the greater part of the floor is formed by the tongue, the remainder being completed by the reflection of the mucous membrane form the sides and under surface of the tongue, to the gum lining the inner aspect of the mandible. The mucous membrane lining the mouth is continuous with the free margin of the lips, and with the mucous lining of the pharynx behind. It is generally of a rose pink tinge and covered by stratified epithelium.

Companies such as Theratech, Inc., for instance, are actively developing systems for the delivery of medicaments via mucosal surfaces. See, for instance, US Patent NO. 5,766,620 for "Buccal delivery of glucagon-like insulinotropic peptides". This patent describes drug delivery systems and methods for administering a glucagon-like insulinotropic peptide to the buccal mucosa for transmucosal drug delivery are described. The drug delivery systems comprise a drug composition containing an effective amount of the glucagon-like insulinotropic peptide and an effective amount of a permeation enhancer for enhancing permeation of glucagon-like insulinotropic peptide through the buccal mucosa and means for maintaining the drug composition in a drug transferring relationship with with buccal mucosa. These systems can be in free form, such as creams, gels, and ointments, or can comprise a device of determined physical form, such as tablets, patches, and troches. A preferred glucagon-like insulinotropic peptide is GLP-1(7-36)amide. The '620 patent describes penetration enhancers selected from the group consisting of an organic solvents, cell-envelope disordering compounds, steroidal detergents, bile salts, chelators, surfactants, non-surfactants, fatty acids, and mixtures thereof.

See also, US Patent No. 5,859,048 ("Pharmaceutics for mucosal administration"), which describes pharmaceutics for mucosal administration containing pharmacologically active peptides or proteins and tolmetin or salts thereof as mucosal absorption enhancers, as well as US Patent Nos. 5,827,525 ("Buccal delivery system for therapeutic agents"); 5,650,192 ("Method for manufacturing buccal delivery device"); 5,849,322 ("Compositions and methods for buccal delivery of pharmaceutical agents"); 5,766,620 ("Buccal delivery of glucagon-like insulinotropic peptides"); 3,948,254 ("Novel drug delivery device"); and 5,726,154 ("Stabilization and oral delivery of calcitonin").

On a related subject, quaternary ammonium salts are commonly used for their preservative and other functions. Benzalkonium chloride, for instance, is a quaternary ammonium salt with antiseptic properties and uses similar to other cationic surfactants. According to Sigma product literature, "the mode of action of quaternary ammonium compounds appears to be associated with the agent's effect on the cytoplasmic membrane, which controls cell permeability." Hence benzalkonium chloride is widely used as a preservative agent in topical, nasal and ocular formulations at concentrations that range from 0.01% to 0.1% for cleansing of wounds and skin.

See also US Patent No. 5,578,567 (assigned to Sandoz), which describes a nasal pharmaceutical composition, comprising hPTH, in a product commercially available as Miacalcin®. The patent describes the optional use of a variety of additives, including preserving agents such as benzalkonium chloride.

On separate subjects, a combination of benzalkonium chloride and a cationic surfactant were found to have an enhancing effect in the iontophoresis of enoxacin, see, for instance, Fang, Intl. J. Pharmacol. 180(2):137-149 (1999), while Woog et al. (US Patent No. 5,503,827) describes a process for the production of multi-dose pharmaceutical preparations containing human protein for infusion or injection, and includes benzalkonium chloride among the various preservatives that can be used in this respect.

Finally, Applicants have developed a unique class of active agents, termed "immunokines", which include bioactive peptides, such as toxins, that have been prepared (e.g., by biosynthetic means) or obtained naturally and rendered inactive, e.g., by ozone treatment, to remove some or all of their disulfide linkages. See, for instance, PCT/US97/08074 (International Publication. No. WO 97/43407).

### SUMMARY OF THE INVENTION

The present invention provides a method and system for administering a macromolecular drug to mucosal surfaces of the buccal cavity (also known as oral cavity). In a preferred embodiment, the method and system include the preparation and delivery of a formulation adapted to contact and adhere to the mucosal tissue of the buccal cavity, and preferably the hard and soft palate of the roof of the mouth. In a particularly preferred embodiment, the delivery formulation comprises an immunokine, as defined herein, in combination with an effective amount of a mucosal absorption enhancer selected from the group consisting of quaternary ammonium salts such as benzalkonium chloride.

In a preferred embodiment, the delivery formulation comprises an effective amount of a mucosal absorption enhancer selected from the group consisting of quaternary ammonium salts, and an effective amount of an inactivated bioactive macromolecule ("immunokine"). e.g., having a molecular weight of at least 500 daltons. The ability to deliver immunokines in this manner, and without the need for complex, expensive, potentially unstable mucosal absorption formulations or devices, greatly facilitates the use of such medicaments.

Applicant has discovered that salts such as benzalkonium chloride have a surprising effect in increasing the absorption of immunokines when delivered to the buccal cavity. This is particularly surprising since benzalkonium chloride is not commonly considered a permeation enhancer, yet in turn, the delivery formulation described herein can be used and is efficacious without the need for other, conventional enhancers or devices. Hence the patient can himself quickly and easily deliver an effective amount of the active agent, with little more that a short aerosol spray into the mouth.

The formulation can be delivered (e.g., by spraying, applying, or device (e.g., patch)) to mucosal surfaces such as the roof of the mouth, or can be delivered topically, e.g., as a liquid, gel or salve. In a preferred embodiment, the formulation is provided in an aerosol container in order to be sprayed onto a mucosal surface within the buccal cavity, e.g., to the roof of the mouth.

### DETAILED DESCRIPTION

Quaternary ammonium salts useful in the system and method of this invention include those commonly used and considered as safe for human use. Such compounds are typically tetrasubstituted ammonium salts in which the substituent groups are preferably hydrocarbon compounds attached to the nitrogen by an N-C bond, and selected from the group consisting of substituted and unsubstituted, saturated and unsaturated, aliphatic and aromatic, and branched and normal chain groups. In all cases the nitrogen atom is pentavalent and is in the positively charged portion of the molecule, thus quaternary ammonium salts are cationic electrolytes. Without intending to be bound by theory, Applicants believe that the efficacy of the present system is due, at least in part, to desirable charge interactions between the quaternary ammonium salt and the polypeptide (particularly, the acidic amino acid residues of the polypeptide). In a preferred embodiment, therefore, the polypeptide of this invention provides a pI of between about 4 and about 8, and preferably between about 5 and about 6, and is used in a formulation of this invention having a pH of between about 8 and about 4, and preferably between about 7 and about S.

Also, and again without intending to be bound by theory, it appears that the use of a quaternary ammonium chloride as a permeation enhancer, in the manner provided herein, provides a variety of unexpected advantages. For instance, it appears that benzalkonium chloride can prevent or lessen the extent of protein aggregation that can otherwise occur in protein formulations of the type described herein. In turn, and particularly when applied to tissue surfaces such as the mucosal surface of the mouth, the dissociation of peptides improves their permeation into the tissue, lessens the effective residence time of the peptides upon the surfaces. In turn, this feature can serve to lessen the potential for immediate, localized immunogenic responses (e.g., swelling, reddening), and in turn, the potential for systemic immunogenic responses. This is particularly surprising in view of the fact that the fleshy tissue of the buccal cavity is known to be prone to localized immunogenic responses to antigens, and that any such response can.be detrimental to the effective delivery of the medicament itself.

A particularly preferred mucosal absorption enhancer of this invention is benzalkonium chloride, also known as alkyl dimethylbenzyl ammonium chloride, alkyldimethyl(phenylmethyl) Quaternary Ammonium Chloride, Ammonyx, and Roccal. BC is commercially available in suitable form from a number of sources, including Sigma Chemical Co. as Product No. B1383.

Quaternary ammonium chlorides are used in an amount effective to increase the permeability and uptake of the immunokine, as compared to a formulation lacking the enhancer. In a preferred embodiment, the QAC is used at a final concentration of between about 0.001 % and about 0.1 %, by weight, and preferably between about 0.005 % and 0.01 %, and more preferably between about 0.004 % and about 0.05 %, based on the weight of the formulation.

The system and method of this invention involve the use of a proteinaceous medicament in the form of an immunokine. The word "immunokine", as used herein, will refer to an inactivated bioactive polypeptide, i.e., a polypeptide that has had some or all of its native tertiary structure altered by the failure to form one or more disulfide linkages. As used in this sense, immunokines are typically natural molecules (either recovered zoom natural sources or synthetically produced) and denatured by exposure to ozone or other oxidizing agents. These denatured molecules lack several functions associated with the native parent molecule and have potential applications in the treatment of numerous diseases, particularly neurological diseases (e.g., multiple sclerosis, amyotrophic lateral sclerosis, viral diseases (e.g., herpes, hepatitis) and cancel. In an alternative embodiment, the inactivated bioactive polypeptide can be one that is genetically engineered to provide substantially all of the native structure and amino acid sequence of an active polypeptide, but in a manner that effectively prevents the formation of one or more of the disulfide bridges formed by the native polypeptide, e.g., by genetically modifying the corresponding cDNA coding for the polypeptide, so as to replace or omit one or more cysteine residues in the resultant translated product. A preferred immunokine, as described herein, exhibits a molecular weight of about 8100 daltons and an isoelectric point of about 4.0.

In one embodiment, the immunokine is prepared by a method comprising the steps of:
a) identifying a polypeptide having a biological activity dependent on the presence of one or more disulfide bridges in its tertiary structure,
b) preparing a cDNA strand encoding the polypeptide,
c) expressing the cDNA under conditions in which the polypeptide is recovered in an inactive form due to the failure to form one or more disulfide bridges, and
d) recovering the inactive polypeptide and formulating it into a composition suitable for parenteral administration to a host.

In a further aspect, the invention provides a method of administering a composition comprising an inactivated bioactive polypeptide to the buccal surfaces of a host, comprising the step of providing the polypeptide in an inactive form and in a composition that includes a quaternary ammonium salt, in order to facilitates the administration of the active to the buccal surfaces of a host. In a related aspect, the invention provides a host having administered such a composition.

In another aspect, the invention provides a composition comprising a bioactive polypeptide that has been rendered inactive by virtue of the failure to form one or more of its disulfide bridges. In a related aspect, the invention provides a composition for *in vivo* administration comprising a bioactive polypeptide that has been inactivated in the manner described herein.

The method can be used to prepare a variety of bioactive polypeptides, including "Group I neurotoxins" (namely, toxins affecting the presynaptic neurojunction), Group II neurotoxins (namely those affecting the postsynaptic neurojunction), and Group III neurotoxins (those affecting ion channels). cDNA sequences for such polypeptides are generally known, or can be determined using conventional techniques.

The cDNA can be expressed using any suitable expression system, under conditions in which the product can be recovered with one or more disulfide bridges unformed. Suitable expression systems include heterologous host systems such as bacteria, yeast or higher eucaryotic cell lines. Examples of useful systems are described, for instance, in "Foreign Gene Expression in Yeast: a Review", Romanos, et al., Yeast, 8:423-488 (1992). See also, "Yeast Systems for the Commercial Production of Heterologous Proteins", Buckholz, et al., Bio/Technology 9:1067-1072 (1991), the disclosures of both Romanos et al. and Buckholz et al. being incorporated herein by reference.

These articles are generally directed at the more common goal of affirmatively *achieving* posttranslational processing and extracellular secretion. Under such conditions, the formation of appropriate disulfide linkages would be included as a necessary step. Given the present description, however, these articles, and the techniques described therein, will be of considerable use to those skilled in the art in achieving the recovery of the unfolded product, e.g., by intracellular expression in yeast.

Preferably, the cDNA is expressed using a yeast expression system, such as *Saccharomyces cerevisiae* and *Pichia pastoris.* More preferably, the cDNA is expressed in a *Pichia* expression system under conditions in which the product is cytoplasmically produced, as opposed to extracellularly secreted. In an exemplary embodiment, the polypeptide is expressed using a *Pichia* expression system, under conditions in which the leader sequence of naturally-occurring cDNA is removed and replaced with only the initiation codon.

Polypeptides of the present invention are generally stable under suitable conditions of storage and use in which the disulfide bonds are prevented from spontaneously reforming, or are allowed to reform in a manner that precludes the undesirable activity of the polypeptide. Optionally, and preferably, once the inactive polypeptide has been recovered, it is treated by suitable means to ensure that the cysteine residues do not spontaneously reform to form disulfide bridges. An example of a preferred treatment means is the use of ozone treatment as described herein. Alternatively, as will be described in greater detail below, ozone treatment can itself be used to selectively break (i.e., oxidize) the disulfide bonds of a native or recombinantly prepared toxin molecule in order provide a stable, inactive form thereof.

In another optional, and alternative, embodiment a polypeptide such as neurotoxin is produced in an inactive form using the *Pichia* expression system described herein.

The delivery method and composition of the present invention provide a unique and valuable tool for the synthesis, recovery and delivery ofbioactive polypeptides in a manner capable of achieving efficacious dosages, while diminishing undesirable activity, yet retaining other useful properties of the polypeptide (such as immunogenicity and antiviral activity).

As used herein, the following words (and inflections thereof) and terms will have the meanings ascribed to them below:
"bioactive" will refer to a polypeptide capable of eliciting at least one biological response when administered *in vivo.*
"polypeptide" will refer to any biomolecule that is made up, at least in part, of a chain of amino acid residues linked by peptide bonds.
"inactive" will refer to a polypeptide that is provided in a form in which at least one form of its bioactive responses is substantially terminated or decreased to a desired extent.
"neurotoxin" will refer to a bioactive polypeptide wherein at least one activity (e.g., binding to the acetylcholine receptor) produces a toxic effect on the nervous system of a mammalian host.

The method of the present invention involves an initial step of identifying a bioactive polypeptide having a tertiary structure in which bioactivity is dependent, at least in part, on the formation of one or more disulfide bridges between cysteine residues. Typically, the polypeptide will be one that is naturally secreted in the course of its synthesis, since it is the secretion process that will provide the necessary posttranslational steps, including disulfide bond formation. Preferably, the polypeptide is one that is stable when recovered and that retains other desirable properties in the unfolded state, such as immunogenicity and/or antiviral, anti-tumor or wound healing activity.

The amino acid sequence and tertiary structure of a number of bioactive polypeptides is known. Suitable polypeptides include those in which one or more disulfide bridges are known to form in the natural configuration, and in which such bridge(s) are necessary for the bioactivity of the polypeptide. Such bridges can be of either an intramolecular (i.e., within a single polypeptide) nature and/or an intermolecular (e.g., between discrete subunits) nature.

Secreted or cell-surface proteins often form additional covalent intrachain bonds. For example, the formation of disulfide bonds between the two -SH groups of neighboring cysteine residues in a folded polypeptide chain often serves to stabilize the three-dimensional structure of the extracellular proteins. Protein hormones such as oxytocin, arginine vasopressin, insulin, growth hormone and calcitonin, all contain disulfide bonds. Enzymes such as ribonuclease, lysozyme, chymotrypsin, trypsin, elastase and papain also have their tertiary structure stabilized by disulfide bonds. Besides the bioactive proteins listed above, there are numerous other proteins that contain disulfide bonds, such as the immunoglobulins (IgA, IgD, IgE, IgM), fibronectin, MHC (major histocompatible complex) molecules and procollagen. Many polypetides from animal venoms also contain disulfide bonds.

In a preferred embodiment, the method of the present invention is used to prepare inactivated forms of neurotoxins, and more preferably neurotoxins from amongst the four groups provided below. As described above, those in Group I typically affect the presynaptic neurojunction, those in Group II typically affect the postsynaptic neurojunction, and those in Group III typically affect ion channels. Lastly, there are also included toxins known only to have a toxic affect by causing membrane damage.

| Neurotoxins | | Membrane-damaging toxins | |
|---|---|---|---|
| Group I | Group II | Group III | |
| notexin | α-conotoxin | dendrotoxins | myotoxins |
| β-bungarotoxin | α-cobrotoxin | scorpion toxins | cardiotoxins |
| crotoxin | erabutoxin | µ-conotoxins | mellitin |
| taipoxin | α-cobratoxin | sea anemone toxins | phospholipases |
| textilotoxin | α-bungarotoxin | omega conotoxins | |
| α-latrotoxin | | apamin (from bee venom) | |
| | | mast cell degranulating peptide | |

The method involves a further step of preparing or isolating a corresponding gene (e.g., a cDNA strand) encoding the polypeptide. Using the primary amino acid sequence discussed above, and in view of the present teaching, those skilled in the art will appreciate the manner in which such polypeptides can be synthesized using genetic engineering techniques. Generally, and preferably, one or more of the native control (e.g., leader) sequences of the desired cDNA are removed and replaced with one or more corresponding sequences in order to facilitate the desired expression.

Polypeptide components from animal venoms, for instance, can be obtained from the animals themselves or from other sources, or they can be created in the laboratory using conventional protein engineering techniques. In the former approach, animals are induced by mechanical or electrical stimuli to release venom from their glands, which travels through a venom canal and out the fang or stinger. The venom is collected and various constituents of the venom are purified by conventional chromatographic techniques.

In the latter approach, constituents from the venom are synthesized by cloning the genes encoding the various polypeptide elements and expressing these genes in heterologous host systems such as bacteria, yeast or higher eucaryotic cell lines. Yeast expression systems are presently preferred, since they tend to provide an optimal combination of such properties as yield and adaptability to human use products.

Expressed products are then purified from any other contaminating host polypeptides by means of chromatographic techniques similar to those used to isolate the polypeptides directly from the venom.

There are significant advantages to the use of host systems other than the venomous animals to obtain the venom components. The danger to human lives in obtaining the venom from the animal is eliminated. There will no longer be a need for the costly animal husbandry required to maintain venomous animals for venom extraction. The quantities of materials that can be obtained from the genetic engineering approach can be one or more orders of magnitude greater than the quantities that can be derived from the venom itself. Moreover, once the gene(s) is cloned and expressed, it can be used to provide a continual, reproducible source in the form of a bacterial, yeast or higher eucaryotic cell line seed culture.

Seed cultures can be stored and transported in the frozen state, lyophilized, or, in some cases, plated on media. Also, the use of genetic engineering tools will enable those skilled in the art to manipulate the genes for the purpose of altering the polypeptide product in any fashion feasible. Using the method of the present invention, in combination with available tools for protein engineering (e.g., site-directed mutagenesis), those skilled will be able to prepare a bioactive polypeptide having any desired level of toxicity, whether non-toxic, or of diminished, equal or greater toxicity than the native form.

The method of the invention provides a further step of expressing the cDNA under conditions in which the polypeptide is recovered in an inactive form due to the failure to form one or more disulfide bridges. As described in greater detail below, this step involves the avoidance of posttranslational processes that would otherwise serve to form such linkages.

Optionally, and preferably, the method provides a further step of treating the inactivated bioactive polypeptides in order to retain the cysteine residues and prevent the spontaneous formation of disulfide bonds. A preferred treatment includes ozone treatment, in the manner described herein. Ozonation affects the cysteine residues by converting the pendent sulfhydryl (-SH) groups to corresponding -SO3X groups, which, unlike the sulfhydryl groups, are unable to form a disulfide bridge. Such treatment is not necessary, however, for those inactivate polypeptides that are found to not spontaneously reform, and that provide the desired activity. Ozonation is preferred for polypeptides such as neurotoxins, where Applicant has shown that upon cleavage and ozonation of the sulfhydryl groups, native neurotoxins are both stable and active.

The invention further includes a delivery formulation comprising a bioactive polypeptide that has been rendered inactive by virtue of the failure to form one or more disulfide bridges. Such polypeptides can be stably stored and used under conditions in which disulfide bonds are prevented from spontaneously reforming.

In yet another aspect, the invention provides a method of administering a bioactive polypeptide to a host, comprising the step of providing the polypeptide in an inactive form and within a suitable composition, and administering the composition to a host. In a related aspect, the invention provides a host having administered such a polypeptide. Compositions of the present invention can be used for a variety of purposes. Compositions are particularly useful in situations calling for a polypeptide in a form that is as close to native as possible, yet without an unwanted bioactivity.

In preparing a delivery formulation of this invention, in a preferred embodiment, the immunokine is formulated in physiological solution such as 0.9% sodium chloride (saline) or buffered saline (e.g., disodium hydrogen phosphate and citric acid) with a pH of between about 4.4 and about 6.5. The immunokine is added to achieve a final concentration effective for its intended use. Typically, for instance where the immunokine is in the form of deactivated alpha cobratoxin, the immunokine is added to a final concentration of between about 100 micrograms/ml (1.28 E-4M) to about 1000 micrograms/ml (1.28E-5M), and preferably between about 500 micrograms/ml and about 700 micrograms/ml.

Benzalkonium chloride (MW 360, and 375 as determined by perchloric acid titration) is described variously as a cationic surfactant (see, e.g., Drug Development Research 40:65-76 (1997) and a cationic disinfectant (J. Orthop. Trauma. 11:121-125 (1997), and is widely used as an antimicrobial agent in pharmaceuticals, particularly opthalmic preparations. The quaternary ammonium salt, e.g., benzalkonium chloride (CAS 8001-54-5) confers upon the immunokine the ability to pass through the mucous membrane comprising the hard and soft palate of the buccal cavity, and into circulating blood.

The delivery formulation is preferably provided within a delivery device, e.g., aerosol and non-aerosol (e.g., pump spray) dispensers as are commonly used for non-fragrance and OTC products. Delivery devices useful in the delivery system of this invention are available from a variety of sources. Representative, and preferred aerosol actuators are available, for instance, from Valois SA in the form of their line of "protruding actuators". The delivery device preferably provides an optimal combination of such features as package design and functionality, stability and ease of use.

In a particularly preferred embodiment, the formulation is applied (e.g., sprayed on) the roof of the mouth in a volume of about 0.1 ml, and preferably between about 0.05 ml and about 0.15 ml.

While the invention has been explained in relation to its preferred embodiments, it is to be understood that various modifications thereof will become apparent to those skilled in the art. The foregoing disclosure is not intended or to be construed to limit the present invention, or to otherwise exclude any such other embodiments, adaptations, variations and equivalent embodiments.

## Claims

1. Use of a delivery formulation comprising an inactivated bioactive peptide comprising a polypeptide that has some or all of its native tertiary structure altered by the failure to form one or more disulfide linkages and an effective amount of a mucosal absorption enhancer selected from the group consisting of quaternary ammonium salts for the manufacture of a medicament for use in buccal cavity administration in the treatment of diseases selected from the group consisting of neurological disease, viral disease and cancer, **characterised in that** the delivery formulation enhances mucosal absorption of the inactivated bioactive peptide in the buccal cavity.

2. The use as claimed in claim 1 wherein the quaternary ammonium salt comprises benzalkonium chloride.

3. The use as claimed in claim 1 or claim 2 wherein the inactivated bioactive peptide comprises an ozone-inactivated toxin.

4. The use as claimed in any of the preceding claims wherein the formulation is delivered by spraying to the roof of a mouth in the buccal cavity.

5. The use as claimed in any of the preceding claims wherein the formulation is provided within an aerosol dispenser.

6. The use as claimed in claim 2 wherein quaternary ammonium chloride is used at a final concentration of between 0.001% by weight and 0.1% by weight based on the weight of the formulation.

7. The use as claimed in claim 2 wherein quaternary ammonium chloride is used at a final concentration of between 0.005% by weight and 0.05% by weight based on the weight of the formulation.

## Patentansprüche

1. Eine Verwendung einer Abgabeformulierung, die ein inaktiviertes biologisch aktives Peptid, das ein Polypeptid beinhaltet, dessen native Tertiärstruktur zum Teil oder ganz durch das Versagen der Bildung einer oder mehrerer Disulfidbindungen geändert ist, und eine effektive Menge eines Schleimhautabsorptionsverstärkers, der aus der Gruppe, bestehend aus quaternären Ammoniumsalzen, ausgewählt ist, beinhaltet, für die Herstellung eines Arzneimittels zur Verwendung bei einer Verabreichung in der Mundhöhle bei der Behandlung von Krankheiten, die aus der Gruppe, bestehend aus neurologischen Krankheiten, Viruskrankheiten und Krebs, ausgewählt sind, **dadurch gekennzeichnet, dass** die Abgabeformulierung die Schleimhautabsorption des inaktivierten biologisch aktiven Peptids in der Mundhöhle verstärkt.

2. Verwendung gemäß Anspruch 1, wobei das quaternäre Ammoniumsalz Benzalkoniumchlorid beinhaltet.

3. Verwendung gemäß Anspruch 1 oder Anspruch 2, wobei das inaktivierte biologisch aktive Peptid ein mit Ozon inaktiviertes Toxin beinhaltet.

4. Verwendung gemäß einem der vorhergehenden Ansprüche, wobei die Formulierung durch Sprühen auf den Gaumen in der Mundhöhle abgegeben wird.

5. Verwendung gemäß einem der vorhergehenden Ansprüche, wobei die Formulierung in einer Aerosolpackung bereitgestellt wird.

6. Verwendung gemäß Anspruch 2, wobei quaternäres Ammoniumchlorid in einer endgültigen Konzentration von zwischen 0,001 Massenanteilen in Prozent und 0,1 Massenanteilen in Prozent auf der Basis der Masse der Formulierung verwendet wird.

7. Verwendung gemäß Anspruch 2, wobei quaternäres Ammoniumchlorid in einer endgültigen Konzentration von zwischen 0,005 Massenanteilen in Prozent und 0,05 Massenanteilen in Prozent auf der Basis der Masse der Formulierung verwendet wird.

## Revendications

1. Utilisation d'une formulation de diffusion comprenant un peptide bioactif inactivé comprenant un polypeptide dont une partie de la structure tertiaire native ou la totalité de celle-ci est altérée faute de pouvoir former une ou plusieurs liaisons disulfures et une quantité efficace d'un stimulateur d'absorption par les muqueuses sélectionné dans le groupe consistant en sels d'ammonium quaternaire pour la fabrication d'un médicament destiné à être utilisé pour être administré dans la cavité buccale dans le traitement de maladies sélectionnées dans le groupe consistant en maladie neurologique, maladie virale et cancer, **caractérisée en ce que** la formulation de diffusion stimule l'absorption par les muqueuses du peptide bioactif inactivé dans la cavité buccale.

2. L'utilisation telle que revendiquée dans la revendication 1 dans laquelle le sel d'ammonium quaternaire comprend du chlorure de benzalkonium.

3. L'utilisation telle que revendiquée dans la revendication 1 ou la revendication 2 dans laquelle le peptide bioactif inactivé comprend une toxine inactivée par l'ozone.

4. L'utilisation telle que revendiquée dans n'importe lesquelles des revendications précédentes dans laquelle la formulation est diffusée par pulvérisation sur la voûte du palais dans la cavité buccale.

5. L'utilisation telle que revendiquée dans n'importe lesquelles des revendications précédentes dans laquelle la formulation est fournie au sein d'un générateur d'aérosol.

6. L'utilisation telle que revendiquée dans la revendication 2 dans laquelle du chlorure d'ammonium quaternaire est utilisé à une concentration finale comprise entre 0,001 % en poids et 0,1 % en poids rapportée au poids de la formulation.

7. L'utilisation telle que revendiquée dans la revendication 2 dans laquelle du chlorure d'ammonium quaternaire est utilisé à une concentration finale comprise entre 0,005 % en poids et 0,05 % en poids rapportée au poids de la formulation.
